# EUROPEAN PATENT APPLICATION

(11) **EP 4 215 180 A1**
(43) Date of publication of application: **26.07.2023**
(21) Application number: 22152789.8
(22) Date of filing: 21.01.2022
(51) Int. Cl.: A61K 9/00, A61K 47/32, A61P 15/18, A61K 31/565, A61K 31/57

(54) **A DRUG DELIVERY SYSTEM**

(71) Applicant: Sever Pharma Solutions, 212 15 Malmö (SE)
(72) Inventor: DE GRAAFF, Wouter, 212 15 Malmö (SE); VAN LAARHOVEN, Hans, 212 15 Malmö (SE)
(74) Representative: Holme Patent A/S

(57) **Abstract**

The present invention relates to a drug delivery system (1) comprising a core (2) made of a first polymeric material (3) and comprising a first active ingredient (4), and a sheath (5) made of a second polymeric material (6) and comprising a second active ingredient (7) dispersed and/or incorporated in the second polymeric material in a concentration of above 10 wt% based on the weight of the sheath (5), and below the percolation threshold of said second active ingredient in the sheath (5). Using the drug delivery system according to the present the inventors have found that it is possible to attain independent and optimal release of two active ingredients without the need for complex assembly of parts and without the need to use sophisticated multi-layer extrusion technology.

## Description

The present invention relates to a drug delivery system, a method of manufacturing said system and the use of said system.

Various types of delivery systems have been developed for the controlled and sustained release of active ingredients preferably by diffusion through the surface of the device.

One such device is the intrauterine device (IUD); Mirena^{®} which is considered one of the safest and most efficient contraception used worldwide. In addition to preventing undesired pregnancies, the device provides several advantages: its use is controlled by the female; it allows for a better regulated dose of drug without attention by the user; and it avoids the destruction (by the intestine and by first pass through the liver) of an appreciable portion of the daily dosage of the drugs compared to their orally delivered counterparts.

Other devices commercially available today are the intra vaginal rings (IVRs), e.g. the Estring^{®}, Femring^{®}, and Nuvaring^{®}, or subdermal contraceptive implants, e.g. the Implanon^{®}, all of which provide controlled and sustained release of steroid molecules over a prolonged period, e.g. several weeks/months.

These known vaginal rings have been found particularly useful for the release of steroids, whose relatively small molecular size and substantially water-insoluble nature permit effective permeation through the hydrophobic polymer, such that therapeutic concentrations may be readily achieved in the body.

However, diffusion in polymers is complex and is known to depend on a number of different factors, e.g. temperature, the manufacturing process, the solubility and diffusivity of the drug in the polymer, the surface area of the drug reservoir, the distance the drug must diffuse through the device to reach its surface and the molecular weight of the drug. Consequently, it remains a challenge to understand, predict and control the diffusion of small and large molecules in polymer systems. In this respect, the use of drug delivery device to deliver drugs requires a design that regulates the release rate so as to reliably provide the user with the appropriate daily dose throughout the lifetime of the device.

In reservoir systems, i.e. a drug loaded core surrounded by a non-medicated membrane/sheath, the drug first partitions into the sheath from the reservoir and then diffuses to the other side of the sheath, where it is taken up by the receiving medium. While the reservoir is saturated, a constant concentration gradient of drug is maintained in the membrane, the rate of drug flux is constant, and zero order release is achieved. However, when drug concentration in the reservoir falls, the gradient across the membrane and the release rate of the drug also decreases.

Furthermore, reservoir systems can be difficult to fabricate reliably, and pinhole defects and cracks in the membrane surrounding the reservoir, can lead to dose dumping, i.e. unintended, rapid drug release over a short period of time.

These problems are avoided in monolithic systems, in which drug is loaded directly into a polymer, which now acts as both a storage medium and a mediator of diffusion. Drug is typically loaded uniformly into monolithic devices, and the release is controlled by diffusion through the monolithic matrix material or through aqueous pores. However, with passing time, release rate decreases, as drug that is deeper inside the monolith device must diffuse to the surface, since it has farther to travel, and the quadratic relation between distance and time becomes important. Since the geometric factor is essential in this respect, the effects can be minimized by using other geometric shapes or hemisphere monoliths to provide near-zero-order release, but such devices are neither easy nor inexpensive to fabricate.

Simultaneous drug delivery/release finds application in a number of different areas. However, the placement of a blend of the drugs in a single delivery device in a proportion equal to the desired delivery rate ratio will almost never achieve the desired result. In many cases, the drugs will not diffuse together through the surface or membrane at the same ratio, as they exist in the blend. The ratio would instead be dependent on the inherent ratio of the normalized permeation rates for the drugs through e.g. a rate-controlling membrane. Flexibility would therefore be limited to the selection of suitable polymer candidates for the sheath. Accordingly, the range of, and degree of control over, the delivery rate ratio, is extremely limited.

Of course the need for maintaining a specified delivery rate ratio can be met by using a separate delivery device for each drug. However, this is clearly undesirable, since the presence of two or more delivery devices will compound the disruption which even a single delivery device might create in the normal physiological activity of an animal or human. In addition, if one delivery device malfunctions, the desired delivery ratio will be lost. Further, complete therapy in a single implantable or insertable delivery device is more acceptable to patients and more efficient to insert and remove. Adjustment of a specified delivery rate can also be met by drug delivery systems comprised of several elements that each release a drug at a specified rate. Examples are two or multi-compartments intravaginal rings, and ring bodies containing drug release capsules. The industrial scale manufacturing of such rings are however complex and expensive.

A further problem with the known devices arranged for releasing more than one drug is that such devices usually show sub-optimum release patterns for the different drugs, whereas it is generally preferred that all drugs are released in a controlled rate during a specified duration of time. Furthermore, since each drug and delivery device combination behaves uniquely, the drug combination could have a significant impact on the drug(s) release characteristics.

Thus, there is a demand for a novel drug delivery system arranged for releasing two active ingredients/drugs in a controlled manner and in the correct ratio, and a method for manufacturing the system that is simple and inexpensive.

Thus, it is a first aspect of the present invention to provide a drug delivery system, which can be loaded with two active ingredients and where each active ingredient is released at a controlled rate independently of the other active ingredient.

In a second aspect according to the present invention is provided a delivery system that reduces the variability of the release rate of active ingredients over time.

In a third aspect according to the present invention a provided a delivery system in which the known problems relating to complicated and expensive manufacturing processes, dose dumping and initial drug burst are eliminated, and which at the same time provides a substantially zero-order release rate of the active ingredient in the core.

In a fourth aspect according to the present invention a provided a delivery system which is stable at room temperature.

In a fifth aspect according to the present invention a provided a delivery system arranged for implantation e.g. subcutaneous or for vaginal or uterine placement in an animal or human.

The novel and unique features whereby these and further aspects are achieved according to the present invention is by providing a drug delivery system comprising a core comprising a one first polymeric material and a first active ingredient, and a sheath comprising a second polymeric material and a second active ingredient dispersed and/or incorporated in the second polymeric material in a concentration of at least 10 wt% based on the weight of the sheath.

The drug delivery system according to the invention relates to a system that comprises a core surrounded, at least partly but preferably completely, by a sheath/membrane. However contrary to the conventional systems of this kind, the sheath of the present invention also comprises a high concentration of an active ingredient, whereby a simultaneous release of two active ingredients is provided.

Within the context of the present invention the term "active ingredient" means a substance intended to be released to a surrounding medium where it will have an effect in the diagnosis, cure, mitigation, treatment or prevention of disease, or an effect in restoring, correcting or modifying physiological functions in a subject, e.g. an animal or human.

Dual administration finds application in a number of different areas, e.g. in contraceptive rings and in dual delivery systems, e.g. implants and rings providing e.g. hormone replacement therapy or contraception and protection against infections, e.g. HIV-infection.

For such dual administration systems it is required to release the two active ingredients simultaneously and at the same time. It is therefore necessary to adjust the release rate of these drugs independently to the physiological optimal rate (mg/day). Using the drug delivery system according to the present invention the inventors have found that it is possible to attain independent and optimal release of two active ingredients without the need for complex assembly of parts and without the need to use sophisticated multi-layer extrusion technology.

Using a drug delivery system in which the concentration of the active ingredient in the sheath is at least 10wt% it is ensured that the desired near zero-order release behavior of the active ingredient in the core are observed for a longer period of time. In addition such high concentrations of active ingredient are associated with good physical stability of the relevant active ingredient.

By substantially zero order is meant that a substantially constant amount of the relevant active ingredient is released over a given period of time. In some embodiments, the system exhibit a substantially zero order release profile of the first active ingredient(s) over a treatment period of at least one month, preferably at least two month, and even more preferred more than three months, or even longer e.g. up to one year.

As discussed earlier it is a well known problem that the release rate of the active ingredient in the core decreases as active ingredient(s) that is deeper inside the core/reservoir must diffuse to the surface. However loading the sheath with a high concentration of active ingredient the inventors of the present invention has found that the release rate of the first active ingredient in the core slightly increases over time, thereby compensating for the further distance said active ingredient must travel to the surface of the delivery system for being released to the surroundings, thereby providing a substantially zero-order relate rate of the first active ingredient during the desired treatment period.

It is important that the second active ingredient is dispersed and/or incorporated in the second polymeric material to an extent sufficient to control the diffusion rate of the first active ingredient through the sheath, while the reduced diffusion rate is still sufficient to maintain an effective level of active ingredient on the surface of the drug delivery system according to the invention.

Without being bound by theory, it is believed that the second active ingredient in the sheath will function as a filler and control the release rate of the first active ingredient. When the second active ingredient present in the sheath is released to the surroundings, the concentration of said second active ingredient is reduced and it is believed that that diffusion of water into the sheath may be facilitated leaving behind an empty porous matrix and/or empty pockets/holes and/or the sheath may collapse thereby ensuring the desired zero-order release profile of the first active ingredient. Thus, it is believed that the space initially occupied by the second active ingredient leaves behind an empty porous matrix which may become water filled due to ingress of water and/or may leave behind empty pockets/holes. This is contrary to the conventional findings in which the release rate of an active ingredient in a core surrounded by a non-medicated sheath slightly decreases over time, i.e. the desired zero order release rate cannot be maintained over a desired treatment period for such conventional systems.

In some embodiments it is preferred that at least 15 wt% of the second active ingredient is dispersed in the second polymeric material, even more preferred at least 20 wt%, and even more preferred at least 25 wt% or even higher. The exact amount of the second active ingredient will depend on the used polymer(s) and active ingredient(s).

The presence of the second active ingredient in the relatively high concentrations will not only lead to an increase in the mean path length the molecules of the first active ingredient have to travel between two points in the sheath, but will also reduce the amount of the first active ingredient which can be dissolved in the polymeric material of the sheath, accordingly decreasing the release rate of the first active ingredient though said sheath. This will provide a reliable release rate and a lower initial burst of the first active ingredient. Accordingly, the sheath can be made smaller, providing a smaller product with a significant lower burst of the first active ingredient.

It is in this respect preferred that the second active ingredient is incorporated and/or dispersed in the second polymeric material in the form of particles, preferably crystals. Such particles/crystals will form a repository of solid, undissolved crystals which will act as seed crystals i.e. as a slow release depot. Over time, when the second active ingredient is delivered to the surroundings, some of the crystals will be unlocked in the second polymeric material, thereby providing a prolonged release of the second active ingredient. Furthermore, the stability of the second active ingredient in the drug delivery system is improved when the active ingredient is incorporated into the sheath as undissolved particles/crystals.

In order to obtained the desired zero order release profile of the first active ingredient during the treatment period, it is preferred that the first active ingredient is dispersed and/or incorporated in the first polymeric material of the core in a concentration of at least 5 wt% of the total weight of the core, preferably at least 10 wt%, preferably at least 15 wt% and even more preferred at least 20 wt%. However, the concentration of the first active ingredient may also be higher such as at least 30 wt% of the of the total weight of the core.

It is preferred that the first active ingredient also is present in the core in the form of particles, preferably crystals, for the same reasons as disclosed for the second active ingredient. In this way the stability of both the first and second active ingredient, and accordingly the drug delivery system according to the intention, is improved.

Alternatively, the first active ingredient can be dissolved in the first polymeric material and in these embodiments the first active ingredient is preferably present in the first polymeric material at a concentration below the saturation concentration of said first active ingredient at a temperature of 25°C.

Since it is expected that some of the first active ingredient will/may redistribute throughout the intravaginal ring upon storage, i.e. the concentration of first active ingredient in the core will drop as a part of the first active ingredient will diffuse into the sheath, the term "below the saturation concentration" refers to the concentration of the first active ingredient in the core measured in an equilibrated drug delivery system, i.e. when an equilibrium of the first active ingredient has been reached in the system.

Without being bound by theory it is believed that maintaining a concentration of the second active ingredient at high concentrations, a porous network path is created by crystals and wherein a number of sites/openings/pores in the matrix of the polymeric material remains empty, ensuring that the active ingredient only can be released through a tortuous path in the sheath, thereby the diffusion length increases and the release rate is controlled.

However, in order to obtain a desired release rate of the first active ingredient it is preferred that the concentration of the second active ingredient in the second polymeric materials does not exceed the percolation threshold of said second active ingredient in the sheath.

Percolation theory can be applied to inert matrix systems in which the sites/openings/pores of the material of the matrix are occupied at random by particles of a specific component. When the particles occupy the neighboring sites in the matrix a cluster is formed, and when this cluster percolates the whole matrix, it is considered to be a sample-spanning cluster i.e. an infinite or percolating cluster. The concentration of component at which there is a maximum probability of appearance of a sample-spanning cluster of this component is named percolation threshold, see Millán M, Caraballo I, Rabasco A.: The role of the drug/excipient particle size ratio in the percolation model for tablets. Pharm Res. 1998;15(2):220―224. Thus, simply speaking, the percolation threshold corresponds to the minimum concentration of the respective active ingredient at which an infinite cluster spans the matrix.

Applied to the present invention, the matrix is formed by the second polymeric material, and when a cluster formed by particles of the second active ingredient provides a continuous phase through the matrix of the sheath, the percolation threshold is reached. It is believed, without being bound by theory, that at or above the percolation threshold the effective diffusion length would decrease, since the cluster of active ingredient will be connected with the surface, and the release rate of the active ingredient would increase.

The percolation threshold depends among others of the particular active ingredient, the polymeric material used, and the size of the core and/or sheath of the drug delivery system according to the invention. However, those skilled in the arts will be able to determine the percolation threshold for any given active ingredient(s) in a drug delivery system according to the present invention in accordance with standard procedures, e.g. the procedures disclosed in e.g. Pharmaceutical Research, Vol. 23, No. 10, October 2006; AAPS PharmSciTech, Vol. II, No.2, June 2010; and/or Pharmaceutica Acta Helvetiae, Vol. 68, issue 1, July 1993, pages 25-33.

In an alternative embodiment the concentration of the concentration of the second active ingredient in the second polymeric materials is 40 wt% or below based on the weight of the sheath, and preferably 35 wt% or below.

In a similar manner the concentration of the first active ingredient in the first polymeric material should be below the percolation threshold of said first active ingredient in the core, or alternatively the concentration of the first active ingredient in the first polymeric materials should be 40 wt% or below based on the weight of the core, e.g. 30 wt% or below.

It must be noted that even though it is known to have small concentrations of active ingredient in a sheath surrounding a drug loaded core, see e.g. WO2013/120888, it is not known to have an active ingredient in the sheath in the high concentrations claimed in the present invention. Small concentrations of active ingredient i.e. concentrations well below 10 wt%, will have no or only a very limited effect on the release rate of the active ingredient in the core, and are accordingly not relevant for the present invention.

A person skilled in the art will based on the context of the present invention understand that it will be possible to control and/or adjust the diffusion rate through the sheath by varying the amount/concentration of the second active ingredient in said sheath, by using different polymeric materials, and/or by using different particle sizes of the second active ingredient or blends of different particles sizes.

It is however preferred that the particles/crystals of the second and optionally the first active ingredient have a mean particle size of between 3 µm and 40 µm, preferably between 8 µm and 24 µm, and even more preferred between 10 and 24 µm as it has been proven that such particles sizes provide the desired near zero order relate rate for a prolongs period of time, i.e. for at desired treatment period of at least one month.

As used herein, the term "crystals" refers to particles of the active ingredient arranged in an ordered microscopic structure, forming a crystal lattice. The term "crystal size" or "particle size" refers to a crystal's or particle's mean particle diameter. Particle size and particle size distribution can be measured using, for example, a Malvern laser scattering particle size analyzer, or any other particle size measurement apparatus or technique known to person's skill in the art. As used herein, the term "crystal size" or "particle size" relates to the particle distribution diameter of the particle. As an example can be mentioned that D90 means that 90% of the particles have a diameter below the given value when measured using laser diffraction.

The polymeric materials used in the drug delivery system of the present invention are preferably suitable for subcutaneous insertion/implantation, or for placement in the uterus or vaginal tract, i.e. the materials are non-toxic and non-absorbable in a patient or animal. In this respect a variety of inert thermoset or thermoplastic elastomer, as well as combinations of polymeric materials are contemplated within the scope of the present invention.

In one embodiment the first and/or second polymeric material is a silicone polymer (thermosetting type). Silicone elastomers, such as poly (dimethylsiloxane) are already used conventionally for IVRs and similar silicones are also contemplated within the scope of the present invention.

It is however preferred that the first and second polymeric material is a thermoplastic polymer, which in principle can be any extrudable thermoplastic polymer material suitable for pharmaceutical use, such as ethylene-vinyl acetate (EVA) copolymers, low-density polyethylene, polyurethanes, and styrene-butadiene copolymers.

In one embodiment, ethylene-vinyl acetate (EVA) copolymer is used as both the first and second polymeric material due to its excellent mechanical and physical properties.

The vinyl acetate concentration of the EVA-polymer determines the rate of diffusion of the active ingredient through the system and generally, the lower the vinyl acetate concentration, the slower the active ingredient will be release from the polymer or migrate through it.

It is in this respect preferred that the second polymeric material of the sheath is a ethylene-vinyl acetate copolymer with a vinyl acetate content from 12 to 28 %, preferably a vinyl acetate content between 14 and 24, such as around 20 %, as these materials will provide the desired release profile through the second polymeric material, i.e. through the sheath.

In order to provide the desired zero order release profile of the first active ingredient it is preferred to have a higher release rate through the core, such that the sheath becomes the rate limiting factor. It is accordingly preferred that the first polymeric material of the core is an ethylene-vinyl acetate copolymer with a vinyl acetate content from 26 to 40 %, preferably 28 %, 33 % or 40 %.

When a specific vinyl acetate content e.g. 20% is mentioned it refers to the content provided by the manufacture. However, manufactures may use different internal analytical methods for determining vinyl acetate content, and there may therefore be variations in the range of 1 - 2 % in the actual vinyl acetate content depending on the manufacture. Thus, in the present invention the vinyl acetate content refers of the vinyl acetate content in the ethylene-vinyl acetate copolymer determined by high resolution NMR according to standard methods.

The drug delivery system according to the invention is a dual-drug delivery system i.e. it comprises two active ingredients. Said active ingredients can in principal be any kind of locally or systematically active medicament, which can be administrated subcutaneously, subdermal, vaginally or to the uterus. It is however preferred that the first active ingredient and/or second active ingredient is selected from a hormone, a steroid, a spermicide, an antimicrobial agent, an anti-viral agent, and combinations of said ingredients.

In a preferred embodiment the first and second active ingredient are different steroids, e.g. different contraceptive agents such as an estrogenic steroid, and/or a progestational steroid. In a preferred embodiment the first active ingredient is estradiol and the second active ingredient is a progestogen selected from the group consisting of levonorgestrel, etonogestrel, d―1―norestrel and norethindrone, preferably levonorgestrel. However the steroids can also be selected in order to treat other conditions, e.g. vaginal atrophy and symptoms associated with menopause, e.g. hot flashes.

In a different embodiment the first and/or second active ingredient may be a spermicides, an antimicrobial agent or an anti-viral agent. Such agents are well known in the art and will not be discussed in greater details in this application.

Irrespectively of the first and second active ingredient or the intended use of the device, the drug delivery system according to the invention is adapted to deliver pharmaceutically effective amounts of active ingredient(s). By "pharmaceutically effective," it is meant an amount, which is sufficient to affect the desired physiological or pharmacological change in the subject. This amount will vary depending upon such factors as the potency of the particular ingredient, the desired physiological or pharmacological effect, and the time span of the intended treatment. Those skilled in the arts will be able to determine the pharmaceutically effective amount for any given active ingredient(s) in accordance with standard procedures.

The thickness of the sheath, which is the outer layer of the drug delivery system according to the present invention, can be varied to further control the release rate of the active ingredients.

In one embodiment the thickness of the sheath is between 0.05 mm and 3 mm. Said thickness is preferably between 0.05 mm and 2 mm, more preferably between 0.1 mm and 2 mm and even more preferably between 0.2 mm and 0.6 mm, depending on the active ingredient(s) and the polymeric material. In certain embodiments the thickness of the sheath is 120 µm, 240 µm or 320 µm.

A person skilled in the art will in view of the present invention understand that a thin sheath can contain less active ingredient than a thicker sheath; and that the concentration of the second active ingredient in the sheath should be enough to sustain release at the desired rate of the relevant active ingredients over the desired treatment period. By using an ethylene-vinyl acetate copolymer grade with higher or lower vinyl acetate-content the sheath's thickness can be altered while maintaining essentially the same average release rate of the first active ingredient. For instance, if a thicker skin is desired because more of the second active ingredient has to be accommodated in the sheath, an ethylene-vinyl acetate copolymer grade with higher vinyl acetate content can be chosen.

In a similar manner the core preferably has a round cross-section with a cross-sectional diameter of between 2 and 8 mm, more preferably between 3 mm and 6 mm and even more preferably around 4 mm.

The drug delivery system according to the invention is preferably in the form of, or formed into, or part of an implant, an intrauterine device or vaginal ring.

In a preferred embodiment, the system of the present technology is an intravaginal ring (IVR). The dimensions of the IVR may vary depending upon the anatomy of the subject, the amount of drug to be delivered to the patient, the time over which the drug is to be delivered, the diffusion characteristics of the drug and other manufacturing considerations. The only requirement being that the IVR should be flexible enough to enable bending and insertion inside the vaginal cavity and rigid enough to withstand the expulsive forces of the vaginal musculature without causing abrasion to the vaginal epithelium. The outer diameter of such IVRs may range, e.g., from about 45 mm to about 65 mm, and/or the length of the fibers forming the IVR may have a length from 150 to 170 mm, preferably from 154 to 160 mm, such as about 157 mm.

In the context of the present invention the term intravaginal ring, also contemplates ring designs or structures, which have other shapes, e.g. polygonal shapes and/or wavy shapes, or where the structure is not a complete and/or closed circle/shape.

In a preferred embodiment according to the present invention the drug delivery system does not comprise more than the two active ingredients, i.e. the first and second active ingredients, and/or does not comprise further cores and/or layers such as sheaths and membranes. Thus in a preferred embodiment the drug delivery system according to the invention consists of a single core and a single sheath completely surrounding said core, and wherein the first active ingredient is part of the core and the second active ingredient is part of the sheath.

The present invention also relates to a method of manufacturing the drug delivery system according to the present invention.

Said method comprises
a. providing a core comprising a first polymeric material and a first active ingredient, and
b. providing a sheath comprising a second polymeric material and a second active ingredient dispersed and/or incorporated in the second polymeric material in a concentration of above 10wt% based on the weight of the sheath, and
c. co-extruding the core and sheath into a fiber.

In order to form the drug delivery device, the fibers obtained in step c. may e.g. be cut into appropriate length, and formed into an implant, an IVR using conventional techniques, or by combining them with other elements for forming an IUD.

As the core and sheath are co-extruded a very simple and inexpensive embodiment according to the invention is provided, however the cores and sheath can be formed in separate injection molding or extrusion steps if preferred. Injection molding and extrusion are well known in the art and will not be discussed further in this application.

It is preferred that the method further comprises a cooling step in which the provided drug delivery system is cooled to a temperature of 20°C or below for providing the crystals in the sheath and optionally the core. This may e.g. be obtained by placing the fiber into a cooling water bath.

Without being bound by theory, the inventors believe that the crystals formed in the sheath and optionally the core is caused by the kinetics of re-crystallization. The relatively high concentrations of active ingredients in both the core and sheath load will result in a higher concentration of "seed" crystals, upon which recrystallization can occur when the fiber is cooled after co-extrusion.

It is preferred that said cooling step is performed immediately after step c., i.e. as fast as is practically possible from a production point of view, i.e. preferably within less than 30 minutes from completion of the fiber in step c.

The invention will be explained in greater detail below, describing an exemplary embodiment of the drug delivery system according to the invention

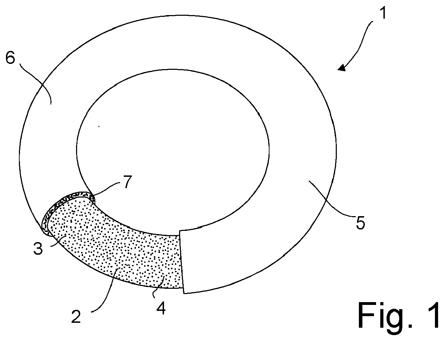

Fig. 1 shows a perspective view of a preferred embodiment of a vaginal ring according to the invention,

The invention is described with the assumption that the drug delivery system is a vaginal ring. However, this assumption is not to be construed as limiting, and the system could just as easily have a different structure/design, e.g. be an IUD, such as a hormone spiral, or an implant.

Fig. 1 shows a preferred embodiment of an intravaginal ring (IVR) 1 according to the invention. In said embodiment the IVR has a reservoir design i.e. it comprises a core 2 made of a first polymeric material 3 and comprising a first active ingredient 4, and a sheath 5 made of a second polymeric material 6 and comprising a second active ingredient 7 dispersed and/or incorporated in the second polymeric material in a concentration of above 10 wt% based on the weight of the sheath.

The drug delivery system according to the invention has a simple and inexpensive design, and can therefore be used equally well both privately and in medical or hospital facilities.

Modifications and combinations of the above principles and designs are foreseen within the scope of the present invention.

## Claims

1. A drug delivery system (1) comprising
- a core (2) comprising a first polymeric material (3) and a first active ingredient (4), and
- a sheath (5) comprising a second polymeric material (6) and a second active ingredient (7) dispersed and/or incorporated in the second polymeric material in a concentration of at least 10 wt% based on the weight of the sheath (5).

2. A drug delivery system (1) according to claim 1, wherein the second active ingredient (7) is dispersed and/or incorporated in the second polymeric material (6) in a concentration of at least 15 wt% preferably at least 20 wt%, and even more preferred at least 25 wt%.

3. A drug delivery system (1) according to claim 1 or 2, wherein the second active ingredient (7) is dispersed and/or incorporated in the second polymeric material (6) in the form of particles, such as crystals.

4. A drug delivery system (1) according to any of the preceding claims, wherein the second active ingredient (7) is dispersed and/or incorporated in the second polymeric material (6) in a concentration below the percolation threshold of said second active ingredient in the sheath (5) .

5. A drug delivery system (1) according to any of the preceding claims, wherein the second active ingredient (7) is dispersed and/or incorporated in the second polymeric material (6) in a concentration of 40 wt% or below based on the weight of the sheath (5), preferably in a concentration of 35 wt% or below.

6. A drug delivery system (1) according to any of the preceding claims, wherein the first active ingredient (4) is dispersed and/or incorporated and/or dissolved in the first polymeric material (3) in a concentration above 5 wt% based on the weight of the core (2), preferably at least 10 wt% based on the weight of the core, preferably at least 15 wt% based on the weight of the core and even more preferred at least 20 wt% based on the weight of the core.

7. A drug delivery system (1) according to any of the preceding claims, wherein the at least one first active ingredient (4) is dispersed and/or incorporated in the first polymeric material (3) in the form of particles, such as crystals.

8. A drug delivery system (1) according to any of the claims 3 to 7, wherein the particles of the second active ingredient (7) and optionally the first active ingredient (4), have a particle size of 3 µm and 40 µm, preferably between 8 µm and 24 µm, and even more preferred between 10 and 24 µm.

9. A drug delivery system (1) according to any of the claims 1 to 6, wherein the first active ingredient (4) is dissolved in the first polymeric material (3) in a concentration below the saturation concentration of said first active ingredient at 25°C.

10. A drug delivery system (1) according to any of the preceding claims, wherein the first polymeric material (3) and/or second polymeric material (6) is at least one inert thermoset or thermoplastic elastomer, such as ethylene-vinyl acetate (EVA) copolymers, low-density polyethylene, polyurethanes, and styrene-butadiene copolymers.

11. A drug delivery system (1) according to any of the preceding claims, wherein the first polymeric material (3) is an ethylene-vinyl acetate copolymer with a vinyl acetate content from 26 to 40%, preferably around 28%, 33% or 40%.

12. A drug delivery system (1) according to any of the preceding claims, wherein the second polymeric material (6) is a ethylene-vinyl acetate copolymer with a vinyl acetate content from 12 to 28%, preferably a vinyl acetate content between 14 and 24%, such as around 20%.

13. A drug delivery system (1) according to any of the preceding claims, wherein the first active ingredient (4) and second active ingredient (7) is selected from a hormone, a steroid, a spermicide, an antimicrobial agent, an anti-viral agent, and combinations of said active ingredients.

14. A drug delivery system (1) according to claim 13, wherein the steroid is a contraceptive agent, e.g. an estrogenic steroid, and/or a progestational steroid.

15. A drug delivery system (1) according to claim 13 or 14, wherein the first active ingredient (4) is a first contraceptive agent, such as estradiol, and the second active ingredient (7) is a different contraceptive agent, such as progesterone.

16. A drug delivery system (1) according to any of the preceding claims, wherein the thickness of the sheath (5) is between 0.05 mm and 3 mm, preferably between 0.05 mm and 2 mm, more preferably between 0.1 mm and 2 mm and even more preferably between 0.1 mm and 0.6 mm.

17. A drug delivery system (1) according to any of the preceding claims, wherein the cross-sectional diameter of the core (2) is from between 2 and 8 mm, more preferably between 3 mm and 6 mm and even more preferably around 4 mm.

18. A method of manufacturing the drug delivery system according to any of the claims 1 - 17, said method comprises
a. providing a core (2) comprising a first polymeric material (3) and a first active ingredient (4),
b. providing a sheath (5) comprising a second polymeric material (6) and a second active ingredient (7) dispersed and/or incorporated in the second polymeric material in a concentration of at least 10 wt% based on the weight of the sheath (5), and
c. co-extruding the core (2) and sheath (5) into a fiber.

19. A method according to claim 18, wherein said method further comprises a cooling step in which the provided drug delivery system is cooled to a temperature of 20°C or below in order to provide crystals of the one second active ingredient (7) in the second polymeric material (6) .

20. A method according to claim 19, wherein said cooling step is performed immediately after step c.

21. A method according to any of the claims 18 to 20, wherein the fiber obtained in step c is cut and/or shaped into a drug delivery device.

22. A dual drug delivery device comprising the drug delivery system according to any of the claims 1 - 17 or obtained with the method according to any of the claims 18 - 21, wherein the drug delivery device is in the form of an implant, an intrauterine device or a vaginal ring.

23. Use of the drug delivery system according to any of the claims 1 - 17, or the dual drug delivery device of claim 22 for providing a substantially zero-order release rate of the at least one first active ingredient (4) for a treatment period of at least 15 days, preferably at least 30 days and even more preferred at least 90 days.
